(19) **Europäisches Patentamt**
**European Patent Office**
**Office européen des brevets**

(11) **EP 1 925 265 B1**

(12) **EUROPÄISCHE PATENTSCHRIFT**

(45) Veröffentlichungstag und Bekanntmachung des Hinweises auf die Patenterteilung:
**06.01.2010 Patentblatt 2010/01**

(51) Int Cl.:
***A61B 19/00*** *(2006.01)*

(21) Anmeldenummer: **06023012.5**

(22) Anmeldetag: **06.11.2006**

(54) **Längenbestimmung eines flexiblen, langen Instruments**

Length determination of a long flexible instrument

Détermination de la longeur d'un instrument longue et flexible

(84) Benannte Vertragsstaaten:
**DE FR**

(43) Veröffentlichungstag der Anmeldung:
**28.05.2008 Patentblatt 2008/22**

(73) Patentinhaber: **BrainLAB AG**
**85622 Feldkirchen (DE)**

(72) Erfinder:
• **Maier, Christian**
**80802 München (DE)**

• **Schaffrath, Claus**
**81377 München (DE)**

(74) Vertreter: **Schwabe - Sandmair - Marx**
**Patentanwälte**
**Stuntzstraße 16**
**81677 München (DE)**

(56) Entgegenhaltungen:
**EP-A- 1 704 825     WO-A-02/36018**
**US-A- 5 921 992**

**Beschreibung**

Die vorliegend Erfindung betrifft die Längenbestimmung eines Instruments, insbesondere eines medizinischen Instruments, insbesondere bei einer klinischen Anwendung, wie zum Beispiel einer Operation. Bei Markernavigationssystemen, insbesondere bei bildgeführter Chirurgie ("image guided surgery"; IGS) ist es wünschenswert, Instrumente zu registrieren und zu kalibrieren. Es sollte also die relative Lage von Teilen des Instruments relativ zu einem Bezugssystem, das beispielsweise im Raum ruht oder das mit einer Detektionseinrichtung (Kamera) verbunden ist, bekannt sein.

Bei manchen Instrumenten ist es nicht möglich oder nicht gewünscht, an der Spitze des Instruments eine Markereinrichtung anzubringen, da beispielsweise die Spitze des Instruments, beispielsweise ein Nagel sonst für die beabsichtigten Zwecke nicht verwendet werden kann. Dennoch ist es in diesem Fall wünschenswert, dass das Markernavigationssystem Informationen über die Lage der Spitze des Instruments liefert. Hierzu ist eine Information über die Länge des Instruments von Nöten. Die Länge des Instruments kann bei starren Instrumenten beispielsweise einfach durch Abgreifen der beiden Enden mittels eines Pointers bestimmt werden. Der Abstand zwischen den Enden entspricht dann der Länge des Instruments. Dies ist jedoch bei nichtideal starren Instrumenten nur ungenau, wobei die Ungenauigkeit mit zunehmender Elastizität des Instruments zunimmt.

US 5,921,922 offenbart ein Verfahren und ein System zum Kalibrieren eines Instruments. Die Kalibrierung wird interoperativ durchgeführt.

WO-A-02/36018 offenbart die Bestimmung einer Verformung eines chirurgischen Instruments. Dabei wird von einer elastischen Biegung des Instruments ausgegangen.

EP-A-1 704 825 offenbart eine Führungsdrahtnavigation. Dabei soll die Position des distalen Endes eines Knochenführungsdrahtes mithilfe eines optischen Tracking-Navigationssystems ermittelt werden.

Aufgabe der Erfindung ist es die Länge flexibler Instrumente vermessen zu können.

Vorstehende Aufgabe wird durch die unabhängigen Ansprüche gelöst. Die abhängigen Ansprüche sind auf vorteilhafte Weiterbildungen gerichtet.

Erfindungsgemäß werden mittels einer Detektionseinrichtung (z. B. Kamera oder Ultraschalldetektor) Markereinrichtungen detektiert. Die Markereinrichtungen umfassen typischerweise drei Marker, die in fester und vorbestimmter relativer Lage zueinander angeordnet sind und insbesondere mechanisch verbunden sind. Die Marker können passive oder aktive Marker sein, wobei die passiven Marker Signale (z.B. Wellen und/oder Strahlung) reflektieren, die in ihre Richtung ausgesendet werden und die aktiven Marker selbst Ursprung der Signale (z.B. Strahlung und/oder Wellen) sind. Die von den (aktiven oder passiven) Markern ausgehenden Signale, bei denen es sich z.B. um Wellensignale oder Strahlungssignale handeln kann, werden von einer Detektionsvorrichtung (z. B. Kamera) detektiert. Um eine Position der Markereinrichtung relativ zu der Detektionseinrichtung festzulegen, wird dabei vorzugsweise die Markereinrichtung bewegt, um der Detektionseinrichtung verschiedene Ansichten der Markereinrichtung zu bieten. Auf dieser Grundlage kann dann in bekannter Weise die relative Lage der Markereinrichtung relativ zu der Detektionseinrichtung, insbesondere in einem im Raum ruhenden Bezugssystem bestimmt werden. In diesem Zusammenhang wird auf die DE 196 39 615 A1 und die entsprechende US-Veröffentlichung 6,351,659 hingewiesen,

Die Lage der Markereinrichtung wird bevorzugt durch die Position der Markereinrichtung in einem vorbestimmen Bezugssystem bestimmt. Vorzugsweise wird als Bezugssystem ein Bezugssystem verwendet, in dem die Detektionseinrichtung ruht. Insbesondere wird die Lage der Markereinrichtung durch die Positionen der Marker, insbesondere der Mittelpunkte der Marker in einem Bezugssystem bestimmt. Die Positionen können zum Beispiel mit kartesischen Koordinaten oder Kugelkoordinaten beschrieben werden. Die relative Lage von einem Teil (z. B. Detektionseinrichtung oder Markereinrichtung) zu einem anderen Teil (z. B. Markereinrichtung) kann insbesondere durch Raumwinkel und/oder Abstände und/oder Koordinaten (in einem Bezugssystem) und/oder Vektoren beschrieben werden und wird vorzugsweise aus dem die Lage beschreibenden Positionen z. B. mittels eines Programms errechnet, das auf einem Computer läuft.

Der hierin verwendete Begriff "relative Lage" oder der Ausdruck «Lage eines Teils A relativ zu einem Teil B" umfasst also den Begriff der relativen Positionen zwischen den zwei Teilen, insbesondere zwischen den Markereinrichtungen und/oder deren Markern oder zwischen einer Markereinrichtung (oder deren Markern) und der Detektionseinrichtung. Insbesondere werden Schwerpunkte oder Mittelpunkte der Teile als punktförmiger Bezugspunkt zum Festlegen einer Position gewählt. Ist die Position eines Teils in einem Bezugssystem bekannt, so kann basierend auf der relativen Lage zweier Teile aus der Position eines der zwei Teile die Position des anderen der zwei Teile berechnet werden.

Insbesondere falls die Markereinrichtung nur zwei Marker umfasst, ist bevorzugt eine Startposition bekannt und die Markersystem erlaubt dann die Verfolgung der Lage der Markereinrichtung bei Bewegung der Markereinrichtung im Raum.

Die Markereinrichtung gemäß der vorliegenden Erfindung umfasst also vorzugsweise mindestens zwei Marker, insbesondere und bevorzugt drei Marker und können natürlich auch mehr als drei Marker umfassen. Die Abmessungen der Marker und die relativen Lagen der Marker zueinander sind bekannt und liegen insbesondere als vorbekannte Daten einer Datenverarbeitungseinrichtung vor. Vorzugsweise ist auch die Form der Marker bekannt.

Weiter umfasst das erfindungsgemäße Markernavigationssystem eine Detektionseinrichtung, die Signale von den mindestens zwei Markern detektiert. Wie zuvor ausgeführt handelt es sich hierbei um von den Markern ausgehende Signale, die entweder aktiv von den Markern ausgesendet werden oder von den Markern reflektiert werden. Im letzteren Fall ist vorzugsweise weiter eine Signalsendequelle beispielsweise eine Infrarotlichtquelle vorgesehen, die Signale (z. B. Ultraschallwellen oder Infrarotlicht) in Richtung auf die passiven Marker (kontinuierlich oder gepulst) aussendet, wobei die passiven Marker die Signale reflektieren. Eine Datenverarbeitungseinrichtung, insbesondere ein Computer erlaubt die Berechnung der relativen Lage der Markereinrichtung relativ zur Detektionseinrichtung, insbesondere die Berechnung der Lage der Markereinrichtung in einem Bezugssystem, indem die Detektionseinrichtung ruht, also beispielsweise in einem Bezugssystem, das in einem Operationssaal ruht.

Die erfindungsgemäße Datenverarbeitungseinrichtung ist ausgebildet, um Berechnungs- und Bestimmungsoperationen durchzuführen. Erfindungsgemäß werden durch die Datenverarbeitungseinrichtung die Lagen der Markereinrichtungen basierend auf den detektierten Signalen, die von den Markereinrichtungen ausgehen, berechnet. Vorzugsweise sind die Gegenstände (Körperstruktur oder Instrument), an denen die Markereinrichtungen angebracht sind, kalibriert. Dies bedeutet, dass die relativen Lagen zumindest zwischen Teilen des Gegenstandes und der Markereinrichtung, die an dem Gegenstand angebracht ist, bekannt sind und/oder insbesondere in der Datenverarbeitungseinrichtung abgespeichert sind, so dass insbesondere Anzeigesignale, die die Lagen der Gegenstände beschreiben, basierend auf den Lagen der Markereinrichtungen bestimmt werden können. Die Lagen der Markereinrichtungen werden bevorzugt relativ zu der Detektionseinrichtung berechnet, also in einem Bezugssystem, in dem die Detektionseinrichtung ruht. Natürlich können die Lagen auch in einem anderen Bezugssystem berechnet werden, z.B. in einem Bezugssystem, in dem der Patient ruht und/oder in dem eine der Markereinrichtungen ruht.

Das erfindungsgemäße Markernavigationssystem erlaubt erfindungsgemäß die Längenbestimmung, vorzugsweise aber auch die Bestimmung des Durchmessers, insbesondere auch der Form des Instruments.

Das Markernavigationssystem umfasst einen Halter, der ausgebildet ist, um das Instrument zu halten. Insbesondere wird ein Ende des Instruments (das zweite Ende) an dem Halter (mechanisch), insbesondere lösbar angebracht. Das Instrument ist so an dem Halter angebracht, dass die relative Lage zwischen dem zweiten Ende des Instruments und dem Halter fixiert ist, d. h. das zweite Ende des Instruments ist relativ zu dem Halter ortsfest. Wie oben ausgeführt, ist dies für das andere Ende, das erste Ende des Instruments nicht gewährleistet, da das Instrument zumindest in einem gewissen Umfang flexibel ist. Bei einem ideal starren Instrument wäre auch das erste Ende des Instruments ortsfest zu dem Halter. Der Halter umfasst vorzugsweise einen Griff und kann zum Beispiel auch eine Maschine, wie beispielsweise ein Bohrgerät umfassen, das ausgebildet ist, das fixierte Instrument zu drehen.

Weiter ist ein Kalibriergerät vorgesehen, das vorzugsweise die Bestimmung des Durchmessers und/oder der Querschnittsform des Instruments erlaubt. Hierzu ist in dem Kalibriergerät vorzugsweise mindestens eine Öffnung vorgesehen. Sind mehrere Öffnungen vorgesehen, so haben diese vorzugsweise eine unterschiedliche Form, sodass unterschiedlich gestaltete Instrumente eingeführt werden können. Vorzugsweise werden Instrumente vermessen, deren Querschnittsform über die gesamte Länge des Instruments konstant ist, denn in die Berechnung der Länge des Instruments fließen Informationen über die Form des Instruments ein, also beispielsweise die Annahme ein, das der Querschnitt des Instruments über die gesamte Länge gleich ist.

Sind in dem Kalibriergerät mehrere Öffnungen vorgesehen, so haben diese vorzugsweise eine unterschiedliche Form und Größe. Auf dieser Art und Weise kann durch passgenaues Einführen eines Instruments in die Öffnungen eine der Öffnungen dem Instrument zugeordnet werden. Da die Form und Größe der Öffnung bekannt ist, entspricht Form und Größe des Querschnitts des Instruments demjenigen der Öffnung. Da weiterhin die relative Lage der Öffnung relativ zu der zweiten Markereinrichtung bekannt ist, die an dem Kalibriergerät angebracht ist, ist weiterhin bekannt, wo sich das erste Ende des Instruments befindet wenn es passgenau, und insbesondere bis zum Anschlag in die Öffnung eingeführt wurde. Da insbesondere auch die relative Lage des Bodens einer jeden Öffnung relativ zu der zweiten Markereinrichtung bekannt ist, ist somit auch die Lage des ersten Endes des Instruments bekannt.

Im Falle eines Instruments mit kreisförmigem Querschnitt, ist die mindestens eine Öffnung ebenfalls eine Öffnung mit einem kreisrunden Querschnitt, also eine zylindrische Bohrung. Natürlich können auch Öffnungen anderer Formen z. B. rechteckige Formen vorgesehen sein, um Instrumente mit rechteckigem Querschnitt passgenau einführen und damit vermessen zu können.

Die oben genannte Detektionseinrichtung ist ausgebildet, um eine erste Lage der ersten Markereinrichtung, die der Halter aufweist und eine zweite Lage der zweiten Markereinrichtung, die das Kalibriergerät aufweist, zu detektieren.

Die oben genannte Datenverarbeitungseinrichtung erlaubt unter der Verwendung bestimmter Informationen, die von der Detektionseinrichtung stammen und/oder vorgegeben sind und/oder eingegeben werden, die Länge des Instruments zu bestimmen, insbesondere zu berechnen.

Bei diesen Informationen handelt es sich insbesondere um die Folgenden. Die oben genannte erste und zweite Lage wurde von der Detektionseinrichtung bestimmt und die Daten hierzu werden in die Datenverarbeitungseinrichtung eingegeben. Die Information über den Querschnitt des Instruments kann von einem Benutzer eingegeben werden, der eine Kennzahl abliest, die neben der Öffnung geschrieben steht, in die das Instrument eingeführt wurde, und die die Form und/oder Größe und/oder Durchmesser der Öffnung kennzeichnet. Alternativ kann dies natürlich beispielsweise auch durch Bildverarbeitung erfolgen, wobei eine Kamera (beispielsweise dieselbe, die auch als Detektionseinrichtung verwendet wird) den Bereich beobachtet, wo das erste Ende des Instruments in die mindestens eine Öffnung des Kalibriergeräts eingeführt wird. Aus der optischen Auswertung des Kamerabildes (beispielsweise den Markierungen neben den Öffnungen), lässt sich dann mittels der Datenverarbeitungseinrichtung ermitteln, in welche Öffnung das erste Ende des Instruments eingeführt ist. Weitere mögliche Arten der Bestimmung der Öffnung, in die das Instrument eingeführt wurde, werden bei der detaillierten Beschreibung weiter unten offenbart. Ist somit die Information über den Querschnitt (beispielsweise der Durchmesser und/oder die Querschnittsfläche und/oder die Form des Querschnitts) des Instruments bekannt, so ist eine wesentliche Information über das Instrument gegeben. Die andere wesentliche Information über das Instrument, nämlich die Länge, wird zusätzlich aus den folgenden Informationen bestimmt. Die Information über die Elastizität des Materials des Instruments. Die Elastizität des Materials des Instruments lässt sich beispielsweise durch den Elastizitätsmodul beschreiben, der auch Youngscher Modul genannt wird. Weiter wird zur Bestimmung der Länge die relative Lage zwischen dem zweiten Ende des Instruments und der ersten Markereinrichtung verwendet. Hierbei kann man nutzen, dass das zweiten Ende des Instruments an einer Anschlagsfläche des Halters anliegend am Halter angebracht wird. Die relative Lage zwischen Anschlagsfläche des Halters und der ersten Markereinrichtung (des Halters) ist bekannt und in der Datenverarbeitungseinrichtung gespeichert. Soweit hierin von »bekannten Daten" oder »bekannten Informationen" oder ähnlichen gesprochen wird, sind dies vorzugsweise Daten, die in der Datenverarbeitungseinrichtung gespeichert sind. Entsprechendes gilt für die relative Lage zwischen der zweiten Markereinrichtung (des Kalibriergerätes) und der mindestens einen Öffnung. Dabei ist, wie oben bereits ausgeführt wurde, insbesondere die relative Lage zwischen dem Boden der mindestens einen Öffnung, an dem das erste Ende des Instruments anliegt und der zweiten Markereinrichtung bekannt.

Zusätzlich zu den oben genannten Informationen ist vorzugsweise auch noch eine Kraft bekannt, die auf das Instrument wirkt. In diesem Fall wird vorzugsweise davon ausgegangen, dass das Instrument, für den Fall, dass keine Kraft wirkt, geradlinig ist und im Falle einer Krafteinwirkung das Instrument sich krummlinig erstreckt, also durch die Krafteinwirkung gebogen wird.

Bei der Berechnung wird von einer stetigen Krümmung ausgegangen, es sollen also keine Knicke im Längsverlauf des Instruments entstehen. Alternativ oder zusätzlich wird davon ausgegangen, dass die Kraft, die auf das Instrument wirkt, nur an den Enden des Instruments wirkt, vorzugsweise nur an einem Ende. Dabei wird bei der Berechnung mittels der Datenverarbeitungseinrichtung vorzugsweise weiter davon ausgegangen, dass die Kraft an dem Ende, an dem sie angreift in die Richtung wirkt, in die sich das Instrument an diesem Ende erstreckt. Die Richtung der Kraft stimmt also mit der Richtung einer Tangente überein, die an dem Ende des Instruments an das gekrümmte Instrument angelegt wird. Insbesondere wirkt eine Kraft zwischen einer Stirnseite des Instruments am zweiten Ende des Instruments und dem Halter. Es wird also davon ausgegangen, dass mit dem Halter auf die Stirnseite des Instruments eine Kraft ausgeübt wird, die zu einer Krümmung des Instruments führt.

Vorzugsweise wird zur Längenbestimmung des Instruments der Halter bewegt, um eine Vielzahl erster und/oder zweiter Lagen zu detektieren und in die Berechnung einfließen zu lassen. Auf diese Art und Weise lässt sich die Genauigkeit der Bestimmung der Länge des Instruments erhöhen, indem beispielsweise die für die verschiedenen ersten und/oder zweiten Lagen bestimmten Längen einer Mittelwertbildung unterzogen werden. Alternativ oder zusätzlich lässt sich aus den detektierten, verschiedenen ersten und zweiten Lagen eine mögliche Auslenkbarkeit des Instruments bei seiner Anwendung bestimmen und vorzugsweise von der Datenverarbeitungseinrichtung abspeichern. Es lässt sich also feststellen, welche möglichen relativen Lagen das erste Ende relativ zum zweiten Ende einnehmen kann. Vorteilhaft ist hierbei, wenn derselbe Benutzer das Verfahren zur Längenbestimmung ausführt, der später das Instrument einsetzt, denn es ist davon auszugehen, dass der Benutzer ähnliche Kräfte aufwendet. Die somit festgestellten möglichen relativen Lagen zwischen dem ersten Ende und dem zweiten Ende können dann bei der (medizinischen) Anwendung des an dem Halter angebrachten Instruments als mögliche Lagen angezeigt werden. Alternativ können diese möglichen Lagen des Instruments, insbesondere des ersten Endes auch berechnet werden, wie im Folgenden ausgeführt wird.

Im Folgenden erfolgt eine detaillierte Beschreibung der Erfindung. Dabei werden weitere vorteilhafte Merkmale der Erfindung offenbart. Verschiedene Merkmale unterschiedlicher Ausführungsformen können miteinander kombiniert werden.

Figur 1 zeigt eine Ausführungsform eines erfindungsgemäßen Markernavigationssystems.
Figur 2 zeigt thematisch die Krümmung eines Instruments, als Grundlage zur Berechnung der Länge.
Figur 3 zeigt möglich Verläufe des Instruments innerhalb einer Körperstruktur.

Detaillierte Beschreibung

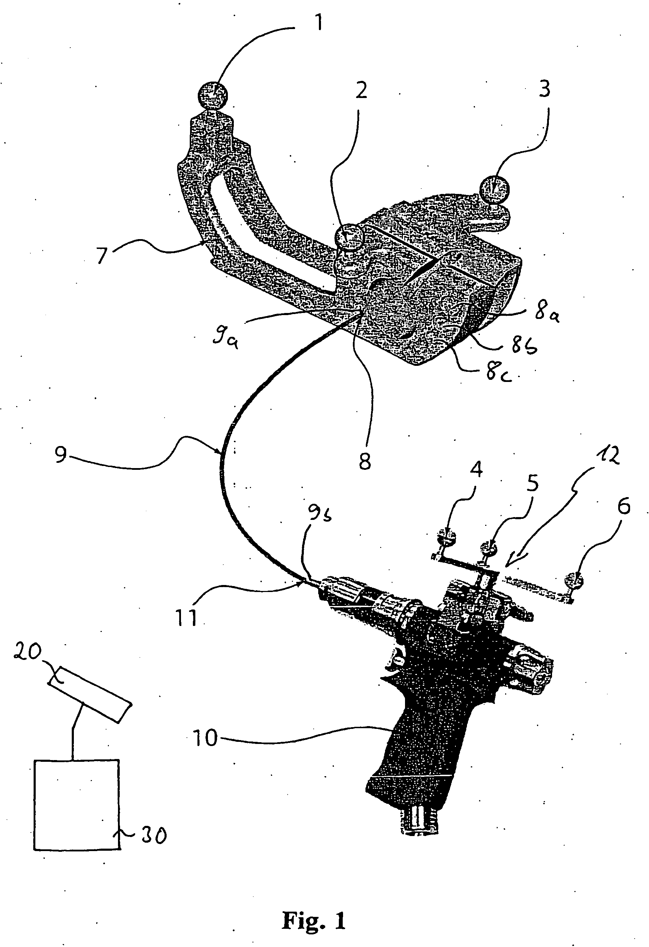

Figur 1 zeigt ein Kalibriergerät 7, ein Instrument 9, einen Halter 10, eine Detektionseinrichtung 20 und eine Datenverarbeitungseinrichtung 30.

Das Kalibriergerät 7 umfasst eine Markereinrichtung, die Markerkugeln 1, 2 und 3 aufweist. Die Markerkugeln 1, 2 und 3 sind über den Körper des Kalibriergeräts 7 miteinander verbunden und nehmen zueinander vorbestimmte relative Lagen ein. Der Körper des Kalibriergeräts 7 umfasst Öffnungen. Von diesen Öffnungen sind nur die Öffnungen 8, 8a, 8b und 8c aus Übersichtlichkeitsgründen mit Bezugszeichen versehen. Die Öffnungen sind vorzugsweise zylindrische Vertiefungen mit einer vorbekannten Tiefe und einem vorbekannten Durchmesser. Vorzugsweise unterscheiden sich die Öffnungen hinsichtlich ihres Durchmessers. Eine Kennzahl, die den Durchmesser der Öffnung bezeichnet ist vorzugsweise neben die Öffnung gedruckt.

Das Instrument 9 ist länglich und flexibel, insbesondere elastisch. Das Instrument hat vorzugsweise einen kreisrunden Querschnitt. In der in Figur 1 gezeigten Abbildung ist das Instrument stark gekrümmt gezeichnet. In der praktischen Anwendung können die Instrumente jedoch erheblich starrer ausgebildet sein und nur eine geringe Krümmung zulassen. Die Instrumente sind insbesondere aus Edelstahl, wie er insbesondere in der Medizin und Chirurgie verwendet wird. Der Elastizitätsmodul von Stahl liegt bei 190 GPa bis 200 GPa. Der Elastizitätsmodul anderer Materialen, wie z. B. Glasfaser liegt bei 50 GPa bis 90 GPa, wohin gegen derjenige von Silikonkautschuk beispielsweise nur 10 MPa bis 100 MPa beträgt.

Das Instrument kann auch andere Durchmesser, wie beispielsweise rechteckförmige Durchmesser, beispielsweise im Falle einer Blechplatte aufweisen.

Beispiele für Instrumente stellen der Kirschnerdraht, Schrauben, insbesondere Schanzschrauben für Knochen, Bohrer, Marknagel, Steinmannpins (für die externe Fixation des Knochens) dar. Typische Durchmesser liegen im Millimeterbereich von z. B. größer 0,1 mm und/oder kleiner 20 mm.

Andere mögliche Instrumente sind die Schenkel einer Zange oder die Klingen einer Schere, die ebenfalls eine gewisse Elastizität aufweisen.

Weitere Beispiele für Instrumente sind Ultraschallsonden, Kanülen, Katheter, insbesondere für die Ventrikeldrainage und zur Verwendung bei der Schmerztherapie (beispielsweise Periduralanästhesie, insbesondere PDK) oder Nadeln, wie sie beispielsweise bei der Vertibroplastie oder Facetteninfiltration verwendet werden.

Das Instrument 9 wird an einem Montagende 11 des Halters 10 an dem Halter 10 befestigt. Beispielsweise wird hierzu das Instrument 9 in eine passend ausgebildete Hülse, die an dem Montageende 11 vorgesehen ist, eingeführt und beispielsweise aufgrund der Passgenauigkeit und/oder mittels einer Schraube mit dem Halter 10 ortsfest verbunden.

Am Halter 10 ist ebenfalls eine Markereinrichtung 12 vorgesehen, die drei Markerkugeln 4, 5 und 6 aufweist. Die Markerkugeln 4, 5 und 6 der Markereinrichtung 12 sind über Arme miteinander so verbunden, dass sie eine vorbestimmte relative Lage jeweils zueinander einnehmen.

Das Kalibriergerät 7 und der Halter 10 sind kalibriert, sodass die relativen Lagen zumindest von Teilen der Oberfläche des Halters 10 und des Kalibriergeräts 7 relativ zu den Markerkugeln bekannt sind.

Insbesondere ist die relative Lage zwischen den Öffnungen (8, 8a, 8b, 8c,...) des Kalibriergeräts 7 relativ zu den Markerkugeln 1, 2 und 3 bekannt. Weiter ist die relative Lage des Montageendes 11 relativ zu der Markereinrichtung 12 (relativ zu den Markerkugeln 4, 5 und 6) bekannt.

Vorzugsweise sind die oben als bekannt bezeichneten Daten und Informationen in der Datenverarbeitungseinrichtung 30 gespeichert. Insbesondere sind die relativen Lagen der Markerkugeln 1, 2 und 3 relativ zu den Öffnungen und die relativen Lagen der Markerkugeln 4, 5 und 6 relativ zu dem Ende 11 in der Datenverarbeitungseinrichtung 30 gespeichert. Weiter gehen in die Datenverarbeitung 30 Datensignale von der Detektoreinrichtung 20 ein. Die Detektoreinrichtung 20 detektiert Signale von den Markerkugeln 1, 2, 3, 4, 5 und 6. Insbesondere ist die Detektionseinrichtung 20 als Kamera ausgebildet, die Licht, beispielsweise Infrarotlicht detektiert, das von den Markerkugeln ausgeht, insbesondere von diesen reflektiert wird. Die Markerkugeln reflektieren Licht, falls sie passive Markerkugeln sind. In diesem Fall können sie beispielsweise durch kontinuierliche oder gepulste Lichtquellen angestrahlt werden, sodass sie das Licht reflektieren.

Die für die Erfindung verwendeten Instrumente sind vorzugsweise dünn, d. h. die Länge beträgt ein Vielfaches des Durchmessers, beispielsweise mehr als das 2-fache oder 5-fache oder 10-fache oder 20-fache des Durchmessers.

Im oben beschriebenen Fall ist die Geometrie des Halters im Computer 30 gespeichert, d. h. die relative Lage zwischen der Markereinrichtung 12 und dem Montageende 11 ist bekannt. Alternativ kann das Montageende 11 beispielsweise in eine der Öffnungen 8, 8a, 8b oder 8c in das Kalibriergeräts gesteckt werden, bis es mit dem Boden der Löcher in Kontakt kommt. Da die Tiefe und Lage (insbesondere Geometrie) der Löcher bekannt ist und die relative Lage zu den Markerkugeln 1, 2 und 3 bekannt ist, kann aus den von der Detektionseinrichtung 20 detektierten und von der Datenverarbeitungseinrichtung 30 weiterverarbeiteten Signalen die relative Lage der Markerkugeln 4, 5 und 6, die ebenfalls detektiert werden, relativ zu dem Montageende 11 von der Datenverarbeitungseinrichtung 30 berechnet werden.

Erfindungsgemäß wird das Instrument 9 in eine vorzugsweise passgenaue Öffnung 8 eingeführt, sodass der Innendurchmesser der Öffnung 8 (zumindest in etwa) mit dem Außendurchmesser des Instruments 9 übereinstimmt. Der Durchmesser der Öffnung 8 kann von dem Kalibriergerät von einer Bedienperson abgelesen werden und in die Datenverarbeitungseinrichtung 30 zur weiteren Verarbeitung eingegeben werden.

Alternativ kann die Feststellung des Durchmessers des Instruments 9 auch automatisch (ohne manuelle Eingabe durch einen Benutzer) erfolgen. Hierzu sind beispielsweise die Öffnungen 8, 8a, 8b, 8c,... weit voneinander beabstandet. Der Abstand ist insbesondere deutlich größer als die Auslenkung, mit der das erste Ende 9a des Instruments 9 von einer Lage abweichen kann, die das erste Ende 9a einnimmt, wenn das Instrument 9 geradlinig ist. Wie bereits oben erwähnt, sind bei Instrumenten aus Stahl nur geringe Auslenkungen von beispielsweise 1 cm zu erwarten. In diesem Fall können dann die vorgenannten Öffnungen einen Abstand von beispielsweise 2 cm aufweisen. Ist dies der Fall, so kann die passende Öffnung aus der relativen Lage zwischen der Markereinrichtung 12 und dem Kalibriergerät 7 abgeleitet werden. Die Datenverarbeitungseinrichtung 30 kann aus dieser relativen Lage bestimmen, dass nur eine der Öffnungen dafür in Frage kommt, dass das Instrument 9 darin eingeführt wurde. Die anderen Öffnungen würden eine erheblich größere Auslenkung des Instruments 9 erfordern, die aber als nicht reell angesehen wird. Wie eine praktisch mögliche Auslenkung des Instruments 9 berechnet wird, wird weiter unten dargelegt.

Die Datenverarbeitungseinrichtung verarbeitet neben der Information über den Durchmesser des Instruments auch noch Information über die Elastizität des Materials des Instruments. Vorzugsweise wird hierzu der Elastizitätsmodul verwendet. Der Elastizitätsmodul ist ein Materialkennwert aus der Werkstofftechnik, der den Zusammenhang zwischen Spannung und Dehnung bei der Verformung eines festen Körpers bei linear elastischem Verhalten beschreibt. Der Betrag des Elastizitätsmoduls ist umso größer, je mehr Widerstand ein Material seiner Verformung entgegensetzt.

Weiter ist die Lage des ersten Endes 9a des Instruments bekannt, denn dieses Ende 9a ist bis zum Anschlag in die dazu passende Öffnung 8 eingeführt. Die Lage des Bodens der Öffnung 8 relativ zu den Markerkugeln 1, 2 und 3 ist ebenfalls bekannt und in der Datenverarbeitungseinrichtung 30 eingespeichert. Durch die Detektion der Markerkugeln 1, 2 und 3 ist somit die Lage des ersten Endes 9a des Instruments 9 bekannt und kann von der Datenverarbeitungseinrichtung weiter verarbeitet werden. Weiter relative Lage des Anschlags für das Instrument in der Hülse 11 des Halters 10 relativ zur Markereinrichtung 12 bekannt. Das Instrument 9 ist ebenfalls bis zum Anschlag in die Hülse 11 (Montageende 11) des Halters 10 eingeführt. Somit ist die relative Lage des zweiten Endes 9b relativ zur Markereinrichtung 12 bekannt. Durch Detektieren der Markereinrichtung 12 mittels der Detektionseinrichtung 20 ist somit die Lage des zweiten Endes 9b bekannt.

Somit sind folgende Daten zur weiteren Verarbeitung in der Datenverarbeitungseinrichtung 30 vorhanden: Der Durchmesser des Instruments 9, die Lage der Enden 9a und 9b (beispielsweise relativ zur Detektionseinrichtung 20 in einem Bezugssystem, indem die Detektionseinrichtung 20 ruht) und das Elastizitätsmodul des Materials des Instruments.

Aus den vorgenannten bekannten Daten wird nun erfindungsgemäß die Länge des Instruments 9 wie folgt berechnet.

Der Elastizitätsmodul E erlaubt die Berechnung der Biegesteifigkeit EI eines langen, dünnen Instruments mit kreisförmigem Querschnitts wie folgt:

$$EI = E * \pi d^4/64 \qquad\qquad (1)$$

Die Differentialgleichung, die den Verlauf bzw. die Krümmung des gebogenen Instruments beschreibt lautet wie folgt:

$$\frac{d^2\vartheta}{dl^2} - \gamma_x \sin\vartheta + \gamma_y \cos\vartheta = 0 \qquad\qquad (2)$$

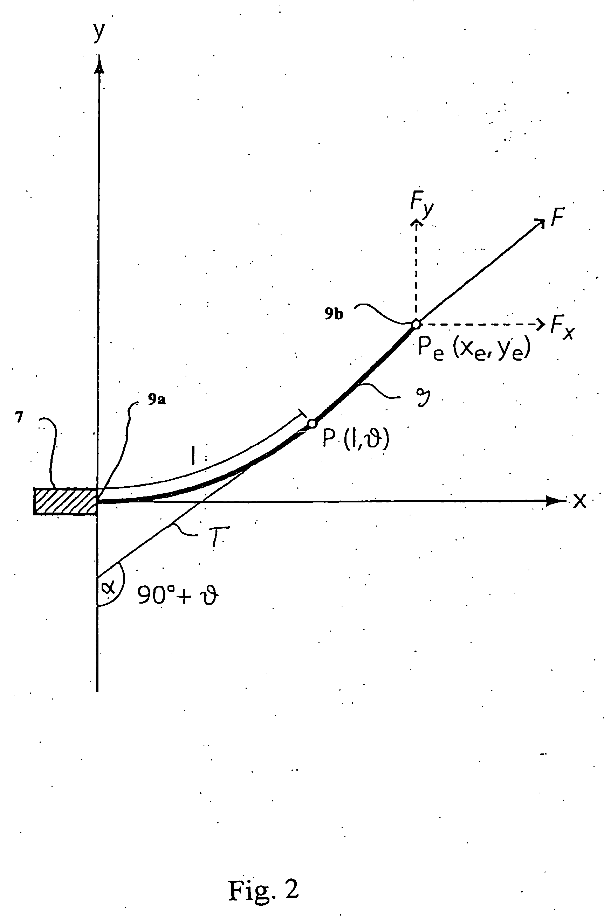

Der Winkel $\vartheta$ ist in Figur 2 erläutert. Der Winkel $\vartheta$ ergibt sich durch Anlegen der Tangente an das gekrümmte Instrument 9 am zweiten Ende 9b und dem Schnittpunkt dieser Tangente T mit der Koordinatenachse y, wobei für den stumpfen Winkel $\alpha$ zwischen der Tangente T und der Koordinatenachse y gilt: $\alpha = 90° + \vartheta$. Der Ursprung des x, y-Koordinatensystems stimmt mit dem Boden in der Öffnung 8 in dem Kalibriergerät 7 überein, wobei das erste Ende des Instruments an dem Boden anliegt.

Für die in der obigen Gleichung (2) erwähnten $\gamma_x$ und $\gamma_y$ gilt:

$$\gamma_x = F_x/EI \qquad\qquad (2a)$$

und

$$\gamma_y = F_y/EI. \qquad (2b)$$

Wie oben erwähnt ist EI die Biegesteifigkeit des Instruments, wobei E das Elastizitätsmodul ist und I von der Geometrie des Instruments abhängt und als Querschnitts-Trägheitsmoment bezeichnet werden kann. Für rechteckförmige Querschnitte ergibt sich beispielsweise ein I, das von dem oben in der Gleichung (1) erwähnten abweicht. Die Größe "1" bezeichnet die Länge von dem ersten Ende 9a zu einem Punkt P. Die Lage des Punkts P lässt sich somit durch die Länge 1 und durch den Winkel $\vartheta$ beschreiben.

Die obige Gleichung (2) kann integriert werden und man erhält dann:

$$\frac{1}{2}\left(\frac{d\vartheta}{dl}\right)^2 + \gamma_x \cos\vartheta + \gamma_y \sin\vartheta = c \qquad (3)$$

Wobei "c" eine Konstante ist und durch die Randbedingung $d\vartheta/dl = \gamma_y X_e$ bestimmt werden kann, die am ersten Ende 9a des Instruments, also am Ursprung des Koordinatensystems gilt. An dem Punkt $P_e$ gilt $\vartheta = \vartheta_e$ und die Länge des Instruments 9 lässt sich wie folgt berechnen:

$$L = \int_0^{\vartheta_e} \frac{d\vartheta}{\sqrt{2\gamma_x + \gamma_y^2 x_e^2 - 2\gamma_x \cos\vartheta - 2\gamma_y \sin\vartheta}} \qquad (4)$$

In der obigen Gleichung sind die Variablen $\vartheta_c$, $\gamma_x$ und $\gamma_y$ unbekannt. Diese drei unbekannten Variablen können durch die folgenden Bedingungen berechnet werden:

$$x_e = \int_0^{\vartheta_e} \frac{\cos\vartheta\, d\vartheta}{\sqrt{2\gamma_x + \gamma_y^2 x_e^2 - 2\gamma_x \cos\vartheta - 2\gamma_y \sin\vartheta}} \qquad (5)$$

$$y_e = \int_0^{\vartheta_e} \frac{\sin\vartheta\, d\vartheta}{\sqrt{2\gamma_x + \gamma_y^2 x_e^2 - 2\gamma_x \cos\vartheta_e - 2\gamma_y \sin\vartheta}} \qquad (6)$$

$$\gamma_x + \frac{1}{2}\gamma_y^2 x_e^2 - \gamma_x \cos\vartheta_e - \gamma_y \sin\vartheta_e = 0 \qquad (7)$$

Bei der letzten Gleichung geht die Randbedingung ein, dass die Krümmung des Instruments oder genauer die Änderung des Winkels $\vartheta$ am Punkt $P_e$, also bei $\vartheta_e = \vartheta_e$ verschwindet.

Sind nun mit Hilfe der obigen Gleichungen (5), (6) und (7) die unbekannten Variablen $\vartheta_e$, $\gamma_x$ und $\gamma_y$ berechnet, so lässt sich mit Hilfe der Gleichung (4) die Länge des Instruments berechnen.

Bei dieser Berechnung wurde berücksichtigt, dass die Biegung des Instruments in einer Ebene erfolgt. Die Koordinaten in der Ebene werden durch das kartesische Koordinatensystem mit den Achsen x und y beschrieben. Die Punkte in der Ebene können darüber hinaus durch die Koordinaten $\vartheta$ und 1 beschrieben werden. Weiter wird angenommen, dass sich das Instrument frei in dem passenden Loch 8 in dem Kalibriergerät 7 drehen kann, sodass keine zusätzlichen Kräfte, insbesondere Verdrillungskräfte entstehen. Man nimmt also an, dass die Verbiegung des Instruments 9 lediglich in der xy-Ebene erfolgt und sonst keine Verformungskräfte wirken.

Die Kraft, die auf das Ende 9b am Punkt $P_e$ wirkt ist die Kraft F, die in zwei Komponenten $F_x$ (also die in x-Richtung wirkende Komponente) und Fy (also die in y wirkende Komponente) zerlegt werden kann. Man nimmt an, dass die Richtung der Kraft F mit der oben beschriebenen Tangente T übereinstimmt. Dies ist eine realistische Annahme, da mit Hilfe des Halters 10 das Instrument 9 in der Praxis beispielsweise in eine Körperstruktur eingeführt werden soll, wobei auf das Instrument 9 in Richtung der Tangente T mit dem Halter 10 eine Druckkraft ausgeübt wird, um das Instrument 9 weiter in der Körperstruktur voranzutreiben. Es wird also davon ausgegangen, dass keine Knickkräfte und Drehmomente auf das Instrument 9 ausgeübt werden, die zu einem Knicken des Instruments 9 am Montageende 11 (also bei $P_e$) führen können.

Bei einer weiteren Ausführungsform der Erfindung ist ein Kraftsensor bei der Halteeinrichtung 10 vorgesehen. Der Kraftsensor ist insbesondere in der Nähe des Montageendes 11. Der Kraftsensor misst vorzugsweise den Betrag der Kraft, der zwischen dem Instrument 9 und dem Halter 10 wirkt. Der Kraftmesser (Kraftsensor) misst insbesondere den Betrag der Kraft und vorzugsweise auch noch die Richtung der Kraft. Ist der Kraftsensor vorgesehen, so kann die Kraft $F_x$ und $F_y$ bestimmt werden. Somit kann aus den obigen Gleichungen (2a) und (2b) $\gamma_x$ und $\gamma_y$ berechnet werden. Dies vereinfacht die Bestimmung der Länge L mit Hilfe der Gleichungen (4) bis (7).

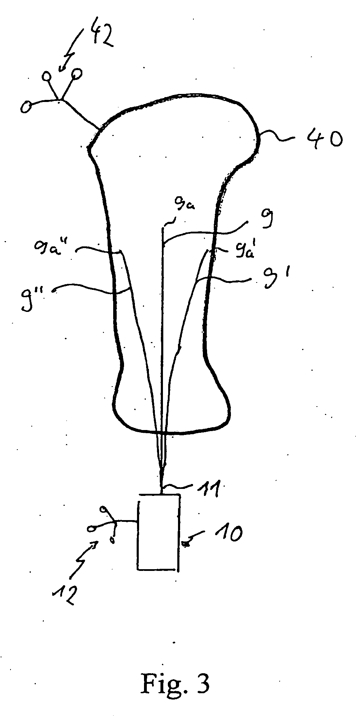

Ist nun die Länge des Instruments bekannt, so kann dies bei der Verwendung des Instruments ausgenutzt werden, um mögliche Positionen des ersten Endes des Instruments anzuzeigen, falls diese unbekannt sind. Man geht also von der Situation aus, dass das zweite Ende an dem Halter 10 angebracht ist und von einem Bediener (Operateur) das Instrument beispielsweise in eine Körperstruktur eingeführt wird. Die Lage des ersten Endes ist dann unbekannt und kann aber wie folgt berechnet werden. Eine bekannte Größe ist nun die Länge L des Instruments. Weiter wird angenommen, dass eine bestimmte Kraft in die Richtung der Längserstreckung des Instruments, also mit einem Winkel $\vartheta = \vartheta_e$ wirkt. Ein Beispiel für eine Situation in der Praxis, bei der mittels des Halters 10 das Instrument 9 in eine Körperstruktur 40 eingeführt wird, ist in Figur 3 gezeigt. Das Instrument 9, beispielsweise ein Kirschnerdraht, wird in eine Körperstruktur (z. B. Knochen) eingeführt. Dabei wird eine typische Kraft F in Längsrichtung des Instruments 9 ausgeübt. Typische Kräfte, die hierin beispielsweise bei der obigen Berechnung zugrunde gelegt werden sind beispielsweise größer als 0,1 N oder 1 N oder 10 N und beispielsweise kleiner als 100 N oder 1.000 N. Eine typische Kraft kann also beispielsweise 1 N betragen. Diese angenommene Kraft hat den Betrag F und wird für die oben genannte Berechnung in die Kräfte $F_x$ und $F_y$ zerlegt, wobei $F_x$ und $F_y$ eine Funktion von F und $\vartheta$ sind. Die Gleichung (4) erlaubt somit die Berechnung von $\vartheta_e$. Somit ist dann über die Gleichungen (5) und (6) die Position $P_e$ und damit die relative Lage zwischen dem ersten Ende und dem zweiten Ende des Instruments berechenbar. Da die Lage des zweiten Endes des Instruments durch Detektion der Markereinrichtung 12 bestimmbar ist, kann hieraus die Lage des ersten Endes des Instruments durch die Datenverarbeitungseinrichtung 30 berechnet werden.

Für eine gegebene Kraft mit Betrag F ergibt sich somit eine mögliche Biegung des Instruments.

Bei dem in Figur 3 gezeigten Fall ist die Länge des Instruments 9 bekannt und wurde beispielsweise mit Hilfe der in Figur 1 gezeigten Anordnung bestimmt. Somit kann mit Hilfe der vorgenannten Gleichungen die mögliche Lage des ersten Endes 9a des Instruments berechnet werden. Die möglichen Lagen sind in Figur 3 als 9a, 9a' und 9a" bezeichnet. Die Linien 9' und 9" bezeichnen den möglichen Verlauf des Instruments innerhalb der Körperstruktur 40. Bei dem in Figur 3 gezeigten Fall erkennt man somit, dass bei einem weiteren vorantreiben des Instruments 9 in die Körperstruktur 40 ein Risiko dahingehend besteht, dass am Ende 9a' die Spitze des Instruments wieder aus der Köperstruktur 40 austreten kann. Dies kann unerwünscht sein.

Die Datenverarbeitungseinrichtung 30 wird deshalb vorzugsweise mit einer Anzeige verbunden, die z. B. die kalibrierte und registrierte Körperstruktur 40 zeigt, die beispielsweise mit einer Markereinrichtung 42 verbunden ist, und/oder einen Konus mit möglichen Positionen des ersten Endes 9a, 9a' und 9a'' und/oder möglichen Verläufen des Instruments gemäß den Linien 9, 9' und 9'' vom ersten Ende bis zum zweiten Ende des Instruments oder einen Abschnitt der möglichen Verläufe. Dem Operateur ist somit ermöglicht, ohne Einsatz eines Röntgengeräts das Risiko zu beurteilen, mit dem Ende 9a in einen unerwünschten Bereich zu kommen. Bei dem in Figur 3 gezeigten Fall, wäre somit eine Überprüfung der Lage des ersten Endes des Instruments 9 angezeigt, da das erste Ende des Instruments droht, die Außenhaut des Knochens 40 zu durchdringen, wenn das Instrument 9 weiter voran getrieben wird. Dies ist leicht von dem Operateur zu überprüfen, das vorzugsweise sowohl die Körperstruktur als auch mögliche Lagen des Instruments durch die Anzeige gezeigt werden.

**Patentansprüche**

1. Markernavigationssystem zur Bestimmung der Länge L flexibler, langer Instrumente (9) insbesondere medizinischer Instrumente, die in ihrer Erstreckung sowohl geradlinig als auch stetig krummlinig sein können, mit einem Halter (10) zum Halten des Instruments (9), das ein erstes und zweites Ende (9a, 9b) aufweist, wobei das zweite Ende (9b) an dem Halter (10) angebracht ist und der Halter (10) eine erste Markereinrichtung (12) aufweist;

mit einem Kalibriergerät (7), das eine zweite Markereinrichtung (1, 2, 3) und mindestens eine Öffnung (8, 8a, 8b, 8c) vorgegebener Form und Tiefe zum Einführen des ersten Endes (9a) aufweist;

mit einer Detektionseinrichtung (20) zum Detektieren einer ersten Lage der ersten Markereinrichtung (12) und einer zweiten Lage der zweiten Markereinrichtung (1, 2, 3);

mit einer Datenverarbeitungseinrichtung (30), **dadurch gekennzeichnet, dass** die Datenverarbeitungseinrichtung ausgebildet ist eine Länge des Instruments (9) entlang dessen geradlinig oder stetig gekrümmten Verlaufs basierend auf der Annahme, dass sich das Instrument frei in der passenden Öffnung (8) in dem Kalibriergerät (7) drehen kann und keine Verdrillungskräfte an dem Instrument (9) anliegen, und Folgendem zu bestimmen:

basierend auf der detektierten ersten und zweiten Lage,
basierend auf Information über den Querschnitt des Instruments (9),
basierend auf Informationen über die Elastizität E des Materials des Instruments (9),
basierend auf der relativen Lage zwischen dem zweiten Ende (9b) und der ersten Markereinrichtung (12), und
basierend auf der relativen Lage zwischen der zweiten Markereinrichtung (1, 2, 3) und der mindestens einen Öffnung (8).

2. Markernavigationssystem nach Anspruch 1, bei welchem die Datenverarbeitungseinrichtung (30) so ausgebildet ist, dass sie die Bestimmung der Länge L basierend auf der Annahme, dass eine vorbestimmte Kraft auf das Instrument (9) einwirkt, oder basierend auf einer gemessenen Kraft, die auf das Instrument (9) am zweiten Ende (9b) einwirkt, vornimmt.

3. Markernavigationssystem nach Anspruch 2, bei welchem die vorbestimmte Kraft, die an mindestens einem der Enden (9a, 9b) des Instruments (9) ansetzt, in die Richtung wirkt, in die sich das Instrument (9) an dem mindestens einen Ende (9a, 9b) erstreckt.

4. Markernavigationssystem nach Anspruch 2 oder 3 bei welchem die Datenverarbeitungseinrichtung (30) die Länge L unter der Annahme berechnet, dass das Instrument (9) durch die Kraft gekrümmt wird.

5. Markernavigationssystem nach einem der vorhergehenden Ansprüche, bei welchem die Information über den Querschnitt umfasst, dass das Instrument (9) einen kreisförmigen Querschnitt mit einem bestimmten Durchmesser hat.

6. Markernavigationssystem nach einem der vorhergehenden Ansprüche, bei welchem die Information über die Elastizität den Elastizitätsmodul E umfasst.

7. Markernavigationssystem nach einem der vorhergehenden Ansprüche, bei welche die Information über die Elastizität des Materials des Instruments (9) umfasst, dass das Material ein Edelstahl ist.

8. Verfahren zur Bestimmung der Länge L eines flexiblen, langen Instruments (9), insbesondere eines medizinischen Instruments, das in seiner Erstreckung sowohl geradlinig als auch krummlinig sein kann, wobei das Verfahren die folgenden Schritte umfasst:

das Instrument (9), das ein erstes und zweites Ende (9a, 9b) aufweist, wird mit seinem zweiten Ende (9b) an dem Halter (10) angebracht, der eine erste Markereinrichtung (12) aufweist, das erste Ende (9a) des Instruments wird in eine Öffnung (8) eines Kalibriergeräts (7) bis zum Anschlag eingeführt, wobei das Kalibriergerät (7) eine zweite Markereinrichtung (1, 2, 3) aufweist und Form und Tiefe sowie die relative Lage der Öffnung (8) relativ zu der zweiten Markereinrichtung (1, 2, 3) bekannt ist; eine erste Lage der ersten Markereinrichtung (12) und eine zweite Lage der zweiten Markereinrichtung (1, 2, 3) wird mittels einer Detektionseinrichtung (20) detektiert; **dadurch gekennzeichnet, dass** mit einer Datenverarbeitungseinrichtung (30) die Länge entlang des geradlinigen oder stetig gekrümmten Verlaufs des Instruments (9) basierend auf folgenden Daten und der Annahme, dass sich das Instrument frei in der passenden Öffnung (8) in dem Kalibriergerät (7) drehen kann und keine Verdrillungskräfte an dem Instrument (9) anliegen, berechnet wird:

der detektierten ersten und zweiten Lage,
Information über den Querschnitt des Instruments (9),
Information über die Elastizität E des Materials des Instruments (9),
der relativen Lage zwischen dem zweiten Ende (9b) und der ersten Markereinrichtung (12) und
der relativen Lage zwischen der zweiten Markereinrichtung (1, 2, 3) und der mindestens einen Öffnung (8).

**Claims**

1. A marker navigation system for determining the length L of long, flexible instruments (9), in particular medical instruments, which can be both linear and constantly curved in their extension, comprising:

   a holder (10) for holding the instrument (9) which comprises a first and second end (9a, 9b), wherein the second end (9b) is attached to the holder (10) and the holder (10) comprises a first marker means (12);
   a calibrating apparatus (7) which comprises a second marker means (1, 2, 3) and at least one opening (8, 8a, 8b, 8c) having a predetermined shape and depth, for inserting the first end (9a);
   a detection means (20) for detecting a first location of the first marker means (12) and a second location of the second marker means (1, 2, 3); and
   a data processing means (30), **characterised in that** the data processing means is designed to determine a length of the instrument (9) along its linear or constantly curved profile, based on the assumption that the instrument can freely rotate in the corresponding opening (8) in the calibrating apparatus (7) and no torque forces act on the instrument (9), and based on the following:

   the detected first and second location;
   information concerning the cross-section of the instrument (9);
   information concerning the elasticity E of the material of the instrument (9);
   the relative location between the second end (9b) and the first marker means (12); and
   the relative location between the second marker means (1, 2, 3) and the at least one opening (8).

2. The marker navigation system according to claim 1, wherein the data processing means (30) is designed such that it determines the length L based on the assumption that a predetermined force acts on the instrument (9) or based on a measured force which acts on the second end (9b) of the instrument (9).

3. The marker navigation system according to claim 2, wherein the predetermined force which acts on at least one of the ends (9a, 9b) of the instrument (9) acts in the direction in which the instrument (9) extends at said at least one end (9a, 9b).

4. The marker navigation system according to claim 2 or 3, wherein the data processing means (30) calculates the length L by assuming that the instrument (9) is curved by the force.

5. The marker navigation system according to any one of the preceding claims, wherein the information concerning the cross-section comprises the information that the instrument (9) has a circular cross-section of a particular diameter.

6. The marker navigation system according to any one of the preceding claims, wherein the information concerning the elasticity comprises the elasticity modulus E.

7. The marker navigation system according to any one of the preceding claims, wherein the information concerning the elasticity of the material of the instrument (9) comprises the information that the material is a high-grade steel.

8. A method for determining the length L of a long, flexible instrument (9), in particular a medical instrument, which can be both linear and curved in its extension, wherein the method comprises the steps of:

   attaching the instrument (9), which comprises a first and second end (9a, 9b), to the holder (10) via its second end (9b), said holder (10) comprising a first marker means (12);
   inserting the first end (9a) of the instrument all the way into an opening (8) of a calibrating apparatus (7), wherein the calibrating apparatus (7) comprises a second marker means (1, 2, 3) and the shape, depth and relative location of the opening (8) relative to the second marker means (1, 2, 3) are known;
   detecting a first location of the first marker means (12) and a second location of the second marker means (1, 2, 3) by means of a detection means (20);
   **characterised in that** the length along the linear or constantly curved profile of the instrument (9) is calculated using a data processing means (30), based on the following data and on the assumption that the instrument can freely rotate in the corresponding opening (8) in the calibrating apparatus (7) and no torque forces act on the instrument (9):
   the detected first and second location;

information concerning the cross-section of the instrument (9);
information concerning the elasticity E of the material of the instrument (9);
the relative location between the second end (9b) and the first marker means (12); and
the relative location between the second marker means (1, 2, 3) and the at least one opening (8).

**Revendications**

1. Système de navigation par repères pour déterminer la longueur L d'instruments (9) longs et flexibles, en particulier d'instrument médicaux, qui peuvent s'étendre aussi bien en ligne droite que constamment en courbe,

   avec une poignée (10) pour tenir l'instrument (9) qui présente une première et une seconde extrémité (9a, 9b), la seconde extrémité (9b) étant placée sur la poignée (10) et la poignée (10) comportant un premier dispositif à repères (12) ;
   avec un appareil de calibrage (7) qui comporte un second dispositif à repères (1, 2, 3) et présente au moins une ouverture (8, 8a, 8b, 8c) de forme et de profondeur prédéfinies pour introduire la première extrémité (9a) ;
   avec un dispositif de détection (20) pour détecter une première position du premier dispositif à repères (12) et une seconde position du second dispositif à repères (1, 2, 3) ;
   avec un dispositif informatique (30), **caractérisé en ce que** le dispositif informatique est conçu pour déterminer la longueur de l'instrument (9) le long de son parcours rectiligne ou constamment courbé, sur la base de l'hypothèse selon laquelle l'instrument peut tourner librement dans l'ouverture (8) ajustée de l'appareil de calibrage (7) et qu'aucune force de torsion ne s'applique sur l'instrument (9), et de ce qui suit :

      sur la base de la première et de la seconde position détectées,
      sur la base de l'information relative à la section transversale de l'instrument (9), sur la base d'informations relatives à l'élasticité E du matériau de l'instrument (9),
      sur la base de la position relative entre la seconde extrémité (9b) et le premier dispositif à repères (12), et
      sur la base de la position relative entre le second dispositif à repères (1, 2, 3) et la au moins une ouverture (8).

2. Système de navigation à repères selon la revendication 1 dans lequel le dispositif informatique (30) est conçu pour déterminer la longueur L sur la base de l'hypothèse selon laquelle une force prédéterminée agit sur l'instrument (9), ou sur la base d'une force mesurée qui agit sur la seconde extrémité (9b) de l'instrument (9).

3. Système de navigation à repères selon la revendication 2 dans lequel la force prédéterminée, qui s'applique sur au moins l'une des extrémités (9a, 9b) de l'instrument (9), agit dans la direction dans laquelle l'instrument (9) s'étend à la au moins une extrémité (9a, 9b).

4. Système de navigation à repères selon l'une quelconque des revendications 2 ou 3 dans lequel le dispositif informatique (30) calcule la longueur L dans l'hypothèse selon laquelle l'instrument (9) est courbé par la force.

5. Système de navigation à repères selon l'une quelconque des revendications 1 à 4 dans lequel l'information relative à la section transversale comprend que l'instrument (9) possède une section de forme circulaire d'un diamètre déterminé.

6. Système de navigation à repères selon l'une quelconque des revendications 1 à 5 dans lequel l'information relative à l'élasticité comprend le module d'élasticité E.

7. Système de navigation à repères selon l'une quelconque des revendications 1 à 6 dans lequel l'information relative à l'élasticité du matériau de l'instrument (9) comprend que le matériau est un acier spécial.

8. Procédé pour déterminer la longueur L d'un instrument (9) long et flexible, en particulier d'un instrument médical, qui peut s'étendre aussi bien suivant une ligne droite que courbée,
   dans lequel le procédé comprend les étapes suivantes :

      l'instrument (9), qui présente une première et une seconde extrémité (9a, 9b), est placé avec sa seconde extrémité (9b) contre la poignée (10) qui comporte un premier dispositif à repères (12),
      la première extrémité (9a) de l'instrument est introduite jusqu'en butée dans une ouverture (8) d'un appareil de calibrage (7), l'appareil de calibrage (7) comportant un second dispositif à repères (1, 2, 3) et la forme, la profondeur ainsi que la position relative de l'ouverture (8) par rapport au second dispositif à repères (1, 2, 3) étant connues ;

une première position du premier dispositif à repères (12) et une seconde position du second dispositif à repères (1, 2, 3) sont détectées au moyen d'un dispositif de détection (20) ;

**caractérisé en ce qu'**on calcule, avec un dispositif informatique (30), la longueur le long du parcours rectiligne ou constamment courbé de l'instrument (9), sur la base des données suivantes et de l'hypothèse selon laquelle l'instrument peut tourner librement dans l'ouverture (8) ajustée de l'appareil de calibrage (7) et aucune force de torsion ne s'applique sur l'instrument :

la première et la seconde position détectées,
l'information relative à la section transversale de l'instrument (9),
l'information relative à l'élasticité E du matériau de l'instrument (9),
la position relative entre la seconde extrémité (9b) et le premier dispositif à repères (12) et la position relative entre le second dispositif à repères (1, 2, 3) et la au moins une ouverture (8).

**Fig. 1**

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5921922 A
- WO 0236018 A
- EP 1704825 A
- DE 19639615 A1
- US 6351659 B